# EUROPEAN PATENT SPECIFICATION

(11) **EP 3 629 879 B1**
(45) Date of publication and mention of the grant of the patent: **16.09.2026**
(21) Application number: 18817006.2
(22) Date of filing: 17.05.2018
(51) Int. Cl.: A47L 15/42

(54) **SENSOR HOUSING**
SENSORGEHÄUSE
BOÎTIER DE CAPTEUR DE MESURE

(30) Priority: 30.05.2017 TR 201707951
(43) Date of publication of application: 08.04.2020
(73) Proprietor: Serdar Plastik Sanayi Ve Ticaret Anonim Sirketi, 06395 Sincan/Ankara (TR)
(72) Inventor: TÜTEK, Serdar, Sincan/Ankara (TR)
(74) Representative: Weickmann & Weickmann PartmbB
(86) International application number: PCT/TR2018/000046
(87) International publication number: WO 2018/231180

(56) References cited:
- EP-A1- 2 634 302
- WO-A1-2007/070086
- WO-A2-2012/140592
- CN-A- 104 695 175
- DE-A1- 102006 041 274
- DE-A1- 102011 102 627
- DE-A1- 102015 100 315

## Description

The present invention relates to a sensor housing which is used in all white appliances such as dishwashers etc. and provides sealing within its structure.

The sensor provides a possibility to measure the water pollution and doesn't require an additional component for a full sealing.

### Technical Field of the Invention

The present invention relates to the housing in which the sensor measuring pollution of water used by the machine is installed and to sealing property of this housing in dishwashers and all similar white appliances.

### State of the Art

There is a sensor measuring the pollution of water in dishwashers and all similar white appliances. The sealing of the housing in which this sensor is installed is provided by an additional O-ring and in this case, there may be leakage because of deformation of the O-ring during mounting or over time. When there is a leakage, firstly the sensor doesn't function properly and goes fully out of order after some time.

DE 10 2011 102627 describes an optical sensor for use in a dishwasher or washing machine. The sensor comprises a housing which is fixed to a wall in the interior. The sensor housing is sealed against the wall with a sealing gasket. It can be a separate visible element or be formed in one piece with the main body of the housing. There is no information on the material of the sealing gasket. The gasket is a conventional annular gasket that provides sealing from two points only.

DE 10 2006 041274 describes a sensor for use in household appliances. The sensor is located in a housing. It can protrude into a liquid in the household appliance, sealed by means of a gasket.

DE 10 2015 100315 describes sealing of a dryer with a drum rotatably mounted in a housing for receiving material to be dried.

### Brief Description of the Invention

The subject matter of the present invention is a sensor housing for a sensor measuring the pollution of water in dishwashers and all similar white appliances, as defined in claim 1.

The gasket provides complete sealing from 4 points including the gasket lower surface and side surface and two sealing tie pieces. It is produced by injecting a second thermoplastic vulcanizate material via 2K injection on the body without requiring any additional component.

### Detailed Description of the Invention

The subject of the invention relates to a pollution sensor housing used in dishwashers and all similar white appliances and determining dirty water. The subject matter of the present invention is a pollution measuring sensor housing as defined in claim 1. In present housings, O- rings attached afterwards for sealing are deformed and lose their sealing capability, whereas the subject matter of the present invention relates to providing complete sealing from 4 points by means of injecting TPV on the housing.

The present invention relates to the housing in which the sensor used in dishwashers and all similar white appliances is installed, and wherein it relates to providing complete sealing by injecting a second thermoplastic via 2K injection on the body without requiring any additional component such as O-ring etc. sealing members.

### Brief Description of the Figures

**Fig. 1****-** is Perspective View of the Pollution Measuring Sensor Housing
**Fig. 2****-** is detailed view of the sealing gasket of the Pollution Measuring Sensor Housing

### List of References

**1-** Turbidity body
**2-** 2K sealing gasket
**3-** Sealing gasket connecting tabs

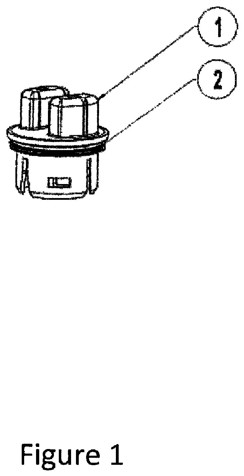

In Fig. 1, assembly view of the pollution measuring sensor is shown. Pollution measuring sensor is installed in the water tank in dishwashers etc. white appliances. The functionality of the sensor housing is to protect the sensor measuring the pollution of water in the tank from the water inside the water tank. The mounting is conducted by the connecting tabs (3) of the water tank engaging the tab housings on the sensor housing (1) shown in the picture.

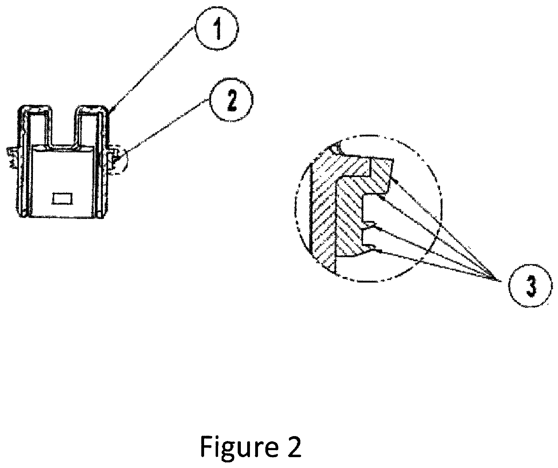

In Fig. 2, detail of the sealing gasket (2), which is produced by 2K injection, of the pollution measuring sensor housing is shown. In existing systems, an additional sealing member such as gasket, O-ring etc. is provided, whereas the present invention provides complete sealing from 4 points with the gasket located within the body. It provides 4 points sealing together with gasket lower surface and side surface that are fitted to the area where it is mounted to the water tank in addition to two sealing tie pieces shown in the Figure.

The sensor housing comprises the body (1) used in dishwashers etc. all white appliance and determining the dirty water and has TPV (soft thermoplastic elastomer) 2K injection (2) injected on this body (1) unlike the existing sealing gaskets. When the body (1) is produced by injection, softer TPV (soft thermoplastic elastomer) is injected on it before removing from the mold thereby obtaining a single piece product made of two different material.

## Claims

1. A sensor housing for a sensor measuring the pollution of water in dishwashers and all similar white appliances, wherein the housing comprises
a body (1) and
a gasket (2), **characterised in that** the gasket (2) comprises two sealing tie pieces and a lower surface and a side surface fitted to an area where the sensor housing is to be mounted, the gasket providing complete sealing from 4 points including the gasket lower surface and side surface and the two sealing tie pieces,
wherein the gasket (2) is made of thermoplastic vulcanisate material and injected on the body (1) via 2K injection, wherein softer thermoplastic vulcanisate material is injected on the body (1) before removing from the mold, thereby obtaining a single piece product made of two different materials.

## Patentansprüche

1. Sensorgehäuse für einen Sensor zum Messen von Verschmutzungen in Wasser von Geschirrspülern und allen ähnlichen Haushaltsgeräten, wobei das Gehäuse einen Körper (1) und
eine Dichtung (2) umfasst, **dadurch gekennzeichnet, dass** die Dichtung (2) zwei Dichtungslaschen und eine Unterseite und eine Seitenfläche, angepasst an den Bereich, an dem das Sensorgehäuse montiert werden soll, umfasst, wobei die Dichtung eine vollständige Abdichtung an 4 Stellen gewährleistet, einschließlich der Unterseite und der Seitenfläche der Dichtung und der zwei Dichtungslaschen,
wobei die Dichtung (2) aus thermoplastischem Vulkanisatmaterial besteht und mittels 2K-Spritzguss auf den Körper (1) aufgespritzt wird, wobei vor dem Entnehmen aus der Form weicheres thermoplastisches Vulkanisatmaterial auf den Körper (1) aufgespritzt wird, wodurch ein einteiliges Produkt aus zwei unterschiedlichen Materialien erhalten wird.

## Revendications

1. Boîtier de capteur destiné à un capteur mesurant la pollution de l'eau dans les lave-vaisselle et tous les appareils électroménagers blancs similaires, dans lequel le boîtier comprend
un corps (1) et
un joint d'étanchéité (2) comprenant deux éléments de fixation d'étanchéité ainsi qu'une surface inférieure et une surface latérale adaptées à une zone où le boîtier de capteur doit être monté, le joint d'étanchéité assurant une étanchéité complète en 4 points, incluant la surface inférieure et la surface latérale du joint d'étanchéité ainsi que les deux éléments de fixation d'étanchéité,
**caractérisé en ce que** le joint d'étanchéité (2) est réalisé en matériau de type vulcanisat thermoplastique et moulé par injection sur le corps (1) selon un procédé d'injection 2K, dans lequel un matériau de type vulcanisat thermoplastique plus souple est injecté sur le corps (1) avant le démoulage, ce qui permet d'obtenir un produit monobloc composé de deux matériaux différents.
